(19) Europäisches Patentamt
European Patent Office
Office européen des brevets

(11) Veröffentlichungsnummer: **0 030 214**
**B1**

(12) **EUROPÄISCHE PATENTSCHRIFT**

(45) Veröffentlichungstag der Patentschrift: **30.01.85**

(21) Anmeldenummer: **80810364.2**

(22) Anmeldetag: **24.11.80**

(51) Int. Cl.$^4$: **C 07 D 213/61,** C 07 D 213/64
// C07D211/80

(54) **Verfahren zur Herstellung von 2,3,5,6-Tetrachlorpyridin und 3,5,6-Trichlorpyridin-2-ol.**

(30) Priorität: **30.11.79 CH 10660/79**

(43) Veröffentlichungstag der Anmeldung:
**10.06.81 Patentblatt 81/23**

(45) Bekanntmachung des Hinweises auf die Patenterteilung:
**30.01.85 Patentblatt 85/05**

(84) Benannte Vertragsstaaten:
**AT BE CH DE FR GB IT LI NL**

(56) Entgegenhaltungen:
**EP-A-0 012 117**

(73) Patentinhaber: **CIBA-GEIGY AG**
**Postfach**
**CH-4002 Basel (CH)**

(72) Erfinder: **Martin, Pierre, Dr.**
**Meisenweg 38**
**CH-4310 Rheinfelden (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 030 214 B1

Courier Press, Leamington Spa, England.

**Beschreibung**

Die vorliegende Erfindung betrifft ein neues Verfahren zur Herstellung von 2,3,5,6-Tetrachlorpyridin und 3,5,6-Trichlorpyridin-2-ol.

Die bisher bekanntgewordenen Verfahren zur Herstellung von 2,3,5,6-Tetrachlorpyridin bzw. 3,5,6-Trichlorpyridin-2-ol sind in verschiedener Hinsicht unbefriedigend. 2,3,5,6-Tetrachlorpyridin kann durch Hochtemperatur-Chlorierung von Pyridin bzw. Pyridinderivaten, wie 3,5-Dichlor-2-trichlormethylpyridin und 2,6-Dichlorpyridin (ca. 200—600, bevorzugt etwa 350—600°C), durch Umsetzung von Glutarsäuredinitril mit Chlor in der Gasphase bei erhöhten Temperaturen (ca. 400—600°C) oder auch durch Chlorierung von ε-Caprolactam oder Cyclohexanon-oxim bei erhöhten Temperaturen erhalten werden. Dabei entstehen jedoch neben dem gewünschten symmetrischen Tetrachlorpyridin eine Reihe anderer hochchlorierter Pyridine, die abgetrennt werden müssen (vgl. z.B. die britischen Patentschriften 1.050.378, 1.334.922 und 991.526, die US Patentschriften 3.420.833 und 3.538.100 sowie die deutschen Offenlegungsschriften 1.445.638 und 2.141.632). 2,3,5,6-Tetrachlorpyridin bzw. 3,5,6-Trichlorpyridin-2-ol können auch durch stufenweise Chlorierung von 6-Brom- oder 6-Chlor-2-äthoxypyridin erhalten werden [vgl. Recueil trav. chim. *70*, 187, (1951)].

Eine andere Möglichkeit besteht darin, dass man die Hochtemperaturchlorierung mit überschüssigem Chorierungsmittel mehr oder weniger selektiv bis zum Pentachlorpyridin führt, und anschliessend das in 4-Stellung befindliche Chloratom entweder mit Zink (vgl. z.B. US Patentschrift 3.993.654) oder elektrolytisch (vgl. z.B. US Patentschrift 3.694.322) abreduziert. Bei der Reduktion mit Zink fallen grosse Mengen von Zinksalzen an, was vom ökologischen Standpunkt aus unerwünscht ist. Die Hochtemperaturchlorierung, die elektrolytische Reduktion sowie die (elektrolytische) Generation von Zink bedingen andererseits einen sehr hohen Energiekonsum. Das Pentachlorpyridin selber weist im übrigen eine relativ starke Haut und Augen reizende Wirkung auf. Das 3,5,6-Trichlorpyridin-2-ol lässt sich durch Hydrolyse von 2,3,5,6-Tetrachlorpyridin herstellen (z.B. Recueil trav. chim. *70*, 182 (1951)).

Aus der britischen Patentschrift 1.024.399 ist ferner bekannt, dass sich Halogenverbindungen, wie Sulfonylhalogenide, Allylhalogenide und Halogennitrile in Gegenwart von Katalysatoren an äthylenisch ungesättigte Verbindungen, wie Olefine mit konjugierten Doppelbindung, Acrylsäure und Acrylsäurederivate, anlagern lassen. Dabei entstehen ausschliesslich offenkettige Produkte.

Es wurde nun ein neues Verfahren gefunden, nach welchem sich 2,3,5,6-Tetrachlorpyridin und 3,5,6-Trichlorpyridin-2-ol unter Vermeidung der obigen Nachteile auf einfache, wirtschaftliche und umweltfreundliche Weise unter Verwendung von leicht zugänglichen, billigen Ausgangsprodukten herstellen lassen.

Das erfindungsgemässe Verfahren besteht darin, dass man Tri-chloracetylchlorid in Gegenwart eines Metalls der Hauptgruppe VIII oder der Nebengruppen VIa, VIIa, Ib und IIb des Periodischen Systems oder einer Verbindung eines solchen Metalls als Katalysator mit Acrylnitril zum 2,3,5,6-Tetrachlorpyridin und/oder 3,5,6-Trichlorpyridin-2-ol umsetzt.

Dabei ist es überraschend, dass durch den Einsatz der ausgewählten erfindungsgemässen Ausgangsmaterialien unter Verschiebung der Chloratome direkt 2,3,5,6-Tetrachlorpyridin bzw. 3,5,6-Trichlorpyridin-2-ol erhalten wird, das heisst, dass unter Verwendung eines Ausgangsproduktes (Trichloracetylchlorid), in welchem drei Chloratome an dasselbe C-Atom gebunden sind, Endprodukte entstehen, in denen sich die drei Chloratome an verschiedenen C-Atomen und in den gewünschten Stellungen (in 3,5- und 6-Stellung) befinden. Dank dieser unerwarteten Reaktion kann auf die aufwendige Hochtemperaturchlorierung verzichtet werden.

Als Katalysatoren für die Anlagerung des Trichloracetylchlorids an das Acrylnitril können im erfindungsgemässen Verfahren an sich bekannte Verbindungen eingesetzt werden, wie Metalle der Hauptgruppe VIII und der Nebengruppen VIa, VIIa, Ib und IIb des Periodischen Systems (gemäss Lehrbuch der anorgan. Chemie, Hollemann-Wiberg, W. de Gruyter & Co., Berlin), z.B. Eisen, Kobalt, Nickel, Ruthenium, Rhodium, Palladium, Chrom, Molybdän, Mangan, Kupfer und Zink. Diese Metalle können in elementarer Form oder in Form von Verbindungen eingesetzt werden. Geeignete derartige Verbindungen sind beispielsweise Oxide, Halogenide, Sulfate, Sulfite, Sulfide, Nitrate, Acetate, Stearate, Citrate, Carbonate, Cyanide und Rhodanide, sowie Komplexe mit Liganden, wie Phosphinen, Phosphiten, Benzoyl- und Acetylacetonaten, Nitrilen, Isonitrilen und Kohlenmonoxid.

Als Beispiele seien genannt: Kupfer(II)oxid, Eisen(III)oxid; Cu(I)-, Cu(II), Fe(II)- und Fe(III)bromide, —jodide und vor allem -chloride, Zinkchlorid, sowie die Chloride des Rutheniums, Rhodiums, Palladiums, Kobalts und Nickels; Cu(II)sulfat, Fe(II)- und Fe(III)sulfat; Cu(II)nitrat und Eisen(III)nitrat; Mangan(III)acetat, Kupfer(II)acetat, Kupfer(II)stearat, Eisen(III)citrat, Cu(I)cyanid; Ruthenium(II)dichloro-tris-triphenylphosphin, Rhodium-dichloro-tris-triphenylphosphin; Chrom- und Nickelacetylacetonat, Kupfer(II)acetylacetonat, Eisen(III)acetylacetonat, Kobalt(II)- und Kobalt(III)acetylacetonat, Mangan(II)acetylacetonat, Kupfer(II)benzoylacetonat; Eisencarbonyl-cyclopentadienylkomplex; Molybdäncarbonylcyclopentadienylkomplex, Chromtricarbonylarylkomplexe, Ruthenium(II)acetatokomplex, Chrom- und Molybdänhexacarbonyl, Nickeltetracarbonyl, Eisenpentacarbonyl, Kobalt- und Mangancarbonyl.

Es können auch Gemische der genannten Metalle mit Metallverbindungen und/oder anderen Zusätzen verwendet werden, wie Kupferpulver in Kombination mit einer der vorerwähnten Kupferverbindungen; Gemische von Kupferpulver mit Lithiumhalogeniden, wie Lithiumchlorid, oder mit Isocyaniden, wie tert.-Butylisocyanid; Gemische von Eisenpulver mit Eisen(III)chlorid, gegebenenfalls unter Zusatz von Kohlenmonoxid; Gemische von Eisen(III)chlorid und Benzoin; Gemische von Eisen(II)- oder Eisen(III)chlorid und Trialkylphosphiten; Gemische von Eisenpentacarbonyl und Jod.

Bevorzugt sind Eisen(II)- und Eisen(III)salze und -komplexe. vor allem Eisen(II)- und Eisen(III)chlorid, sowie Eisenpulver; Ruthenium(III)chlorid, Ruthenium(II)dichlor-tris-triphenylphosphin, Kupferpulver, Kupferbronze, Kupfer(I)- und Kupfer(II)salze und -komplexe, wie Cu(I)-chlorid, Cu(II)chlorid, Cu(I)-bromid, Cu(II)bromid; Cu(II)acetat, Cu(II)acetylacetonat, Cu(II)benzoylacetonat, Cu(II)sulfat, Cu(II)nitrat, Cu(I)cyanid und Cu(I)jodid.

Ganz besonders bevorzugt sind Kupferpulver, Kupferbronze, Kupfer(I)- und Kupfer(II)chlorid bzw. -bromid, Kupfer(I)jodid, sowie deren Gemische.

Die Katalysatoren werden im allgemeinen in Mengen von etwa 0,01 bis 10 Mol-%, bevorzugt 0,1 bis 5 Mol-%, bezogen auf das Acrylnitril, verwendet.

Die Umsetzung des Trichloracetylchlorids mit dem Acrylnitril wird zweckmässig in Gegenwart eines organischen Lösungsmittels vorgenommen.

Geeignete organische Lösungsmittel sind solche, in denen die Katalysatoren ausreichend löslich sind oder die mit den Katalysatoren Komplexe bilden können, die aber gegenüber dem Trichloracetylchlorid und dem Acylnitril inert sind. Beispiele solcher Lösungsmittel sind Alkylnitrile, besonders solche mit 2—5 C-Atomen, wie Acetonitril, Propionitril und Butyronitril; 3-Alkoxypropionitrile mit 1 oder 2 C-Atomen im Alkoxyteil, wie 3-Methoxypropionitril und 3-Aethoxypropionitril; aromatische Nitrile, vor allem Benzonitril; ferner auch Acrylnitril (d.h. überschüssiges Reagens als Lösungsmittel); aliphatische Ketone mit bevorzugt insgesamt 3—8 C-Atomen, wie Aceton, Diäthylketone, Methylisopropylketon, Diisopropylketon, Methyl-tert-butylketon; Alkyl- und Alkoxyalkylester von aliphatischen Monocarbonsäuren mit insgesamt 2—6 C-Atomen, wie Ameisensäuremethyl- und -äthylester, Essigsäuremethyl-, -äthyl-, -n-butyl und -isobutylester sowie 1-Acetoxy-2-methoxyäthan; cyclische Aether, wie Tetrahydrofuran, Tetrahydropyran und Dioxan; N,N-Dialkylamide von aliphatischen Monocarbonsäuren mit 1—3 C-Atomen im Säureteil, wie N,N-Dimethylformamid, N,N-Dimethylacetamid, N,N-Diäthylacetamid und N,N-Dimethylmethoxyacetamid; Aethylenglykol- und Diäthylenglykoldialkyläther mit je 1—4 C-Atomen in den Alkylteilen, wie Aethylenglykoldimethyl-, -diäthyl- und -di-n-butyläther; Diäthylenglykoldiäthyl- und -di-n-butyläther; Hexamethylphosphorsäuretriamid (Hexametapol).

Besonders bevorzugte Lösungsmittel sind Alkylnitrile mit 2—5 C-Atomen und 3-Alkoxypropionitrile mit 1 oder 2 C-Atomen im Alkoxyteil, besonders Acetonitril, Butyronitril, 3-Methoxypropionitril und Acrylnitril.

Bevorzugt erfolgt die Umsetzung zum 2,3,5,6-Tetrachlorpyridin und/oder 3,5,6-Trichlorpyridin-2-ol bei einer Temperatur zwischen etwa 70 und 220°C, insbesondere zwischen 130 und 200°C. Dabei kann in offenem oder geschlossenem Gefass gearbeitet werden. Besonders bevorzugt wird die Reaktion in geschlossenem System bei einem der jeweiligen Reaktionstemperatur entsprechenden Druck, der z.B. im Bereich von 0 bis 50 bar liegen kann, durchgeführt.

Beim erfindungsgemässen Verfahren entstehen im allgemeinen Gemische von 2,3,5,6-Tetrachlorpyridin und 3,5,6-Trichlorpyridin-2-ol. Durch Variation der Reaktionsbedingungen lässt sich die Reaktion jedoch selektiv steuern. Das 2,3,5,6-Tetrachlorpyridin kann auch nachträglich nach an sich bekannten Methoden zum 3,5,6-Trichlorpyridin-2-ol hydrolysiert werden, das sich seinerseits leicht wieder in das 2,3,5,6-Tetrachlorpyridin überführen lässt [vgl. z.B. Recueil, *51*, 182 und 187 (1951)].

Für eine selektive Reaktionsführung zugunsten der Bildung von 2,3,5,6-Tetrachlorpyridin sind erhöhte Temperaturen (oberhalb etwa 140°C) und das Arbeiten in geschlossenem System, gegebenenfalls unter Zusatz von HCl-bildenden Mitteln, wie Phosgen, Aluminiumtrichlorid, Trialkylammoniumchloride, $POCl_3$ und $PCl_5$, besonders bevorzugt. Andererseits kann man das Reaktionsgemisch auch nachträglich mit HCl oder HCl-abspaltenden Mitteln, wie Phosgen, $POCl_3$, $PCl_5$ oder $CH_3P(O)Cl_2$, behandeln. Durch Nachbehandlung des Reaktionsgemisches mit Hydroxylionen liefernden Mitteln, wie Gemischen von Wasser und NaOH, $NaHCO_3$, $Na_2CO_3$ oder tertiären Aminen, wie Triäthylamin, Tributylamin, Pyridin etc., erhält man ausschliesslich das 3,5,6-Trichlorpyridin-2-ol.

Bei der erfindungsgemässen Umsetzung kann intermediär auch das 3,3,5,6-Tetrachlor-3,4-dihydropyridon-(2) gebildet werden.

Das 2,3,5,6-Tetrachlorpyridin und 3,5,6-Trichlorpyridin-2-ol können auf übliche Weise isoliert und gegebenenfalls gereinigt werden, z.B. durch Kristallisation, Destillation, Sublimation, Chromatographie und besonders durch Wasserdampfdestillation.

Das 2,3,5,6-Tetrachlorpyridin bzw. 3,5,6-Trichlorpyridin-2-ol sind bekannte Verbindungen und eignen sich zur Herstellung verschiedener Wirkstoffe, vor allem zur Herstellung von Insektiziden, Herbiziden und Fungiziden [vgl. z.B. US Patentschriften 4.133.675, 3.244.586, 3.355.278 und 3.571.421; französische Patentschrift 2.171.939 sowie J.Agr.Food Chem. *14*, 304 (1966)].

Beispiel 1:

181,8 g Trichloracetylchlorid, 53,1 g Acrylnitril, 4 g Kupfer(I)chlorid und 400 ml Acetonitril werden 2 Stunden bei 180°C in einem 1 Liter Email-Autoklaven gehalten. Anschliessend wird das Reaktionsgemisch eingedampft. Der Rückstand wird mit heissem n-Pentan extrahiert. Der eingedampfte kristalline Extrakt wird mit Methanol digeriert. Die anfallenden Kristalle (2,3,5,6-Tetrachlorpyridin; Smp. 90—91°C) werden abfiltriert. Die methanolische Lösung wird eingedampft, und der Rückstand wird aus n-Hexan kristallisiert. Man erhält 3,5,6-Trichlorpyridin-2-ol; Smp. 170—171°C.

Beispiel 2:

Man verfährt wie in Beispiel 1 angegeben. Nach dem Abtrennen des 2,3,5,6-Tetrachlorpyridins wird eine Destillation mit überhitztem Wasserdampf durchgeführt. Man erhält 3,5,6-Trichlorpyridin-2-ol in Form schneeweisser Kristalle; Smp. 168—170°C. $^1H$—NMR—Spektrum ($DMSO-d_6/D_2O$) in ppm: 8,07(s).

Beispiel 3:

Wird die in Beispiel 1 beschriebene Reaktion bei 115°C durchgeführt und mehrmals unterbrochen, um Proben zu entnehmen, so kann das 3,3,5,6-Tetrachlor-3,4-dihydropyridon-(2) der Formel

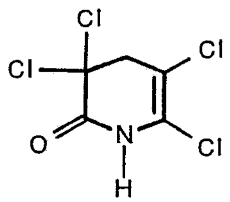

als Zwischenprodukt nachgeweisen bzw. isoliert werden; Smp. 127—128°C. Das 3,3,5,6-Tetrachlor-3,4-dihydropyridon-(2) fällt beim Abkühlen aus und verschwindet im weiteren Reaktionsverlauf wieder. IR—Spektrum ($CHCl_3$) in $cm^{-1}$: 1720(CO), 1665 (C=C). $^1H$—NMR—Spektrum ($CDCl_3/D_2O$) in ppm: 3,57(s).

Beispiel 4:

Das in Beispiel 1 beschriebene Reaktionsgemisch wird 8 Stunden bei 140°C gehalten. Das eingedampfte Reaktionsgemisch wird direkt einer Wasserdampfdestillation unterworfen. Man erhält weisse Kristalle, die laut spektroskopischen Daten (IR- und $^1H$—NMR—Spektrum) aus einem Gemisch von 2,3,5,6-Tetrachlorpyridin und 3,5,6-Trichlorpyridin-2-ol bestehen.

Beispiel 5:

Beispiel 4 wird wiederholt, mit dem Unterschied, dass das rohe eingedampfte Reaktionsgemisch in Aethanol aufgenommen und solange mit 10% iger Natronlauge versetzt wird, bis die Lösung alkalisch bleibt. Nach dem Abdampfen des Aethanols wird mit Wasserdampfdestilliert. Man erhält reines 3,5,6-Trichlorpyridin-2-ol; Smp. 170—172°C.

Beispiel 6:

Beispiel 4 wird wiederholt, mit dem Unterschied, dass man das eingedampfte Reaktionsgemisch mit der gleichen Gewichtsmenge $CH_3P(O)Cl_2$ versetzt und 2,5 Stunden auf 180°C (Badtemperatur) erhitzt. Nach dem Abkühlen wird vorsichtig mit Eiswasser zersetzt und anschliessend mit Wasserdampf destilliert. Man erhält ausschliesslich 2,3,5,6-Tetrachlorpyridin; Smp. 90°C.

Beispiel 7:

Das gemäss Beispiel 4 erhaltene Reaktionsgemisch wird unter leichtem HCl-Gegenstrom durch ein auf 180°C erhitztes Rohr geführt. Das erhaltene Pyrrolisat wird mit Wasserdampf destilliert. Man erhält das 2,3,5,6-Tetrachlorpyridin (Smp. 89—91°C) in Form weisser Schuppen.

**Patentansprüche**

1. Verfahren zur Herstellung von 2,3,5,6-Tetrachlorpyridin und/oder 3,5,6-Trichlorpyridin-2-ol, dadurch gekennzeichnet, dass man Trichloracetylchlorid in Gegenwart eines Metalls der Hauptgruppe VIII oder der Nebengruppen VIa, VIIa, Ib und IIb des Periodischen Systems oder einer Verbindung eines solchen Metalls als Katalysator mit Acrylnitril umsetzt.

2. Ein Verfahren nach Anspruch 1, dadurch gekennzeichnet, dass man die Umsetzung des Trichloracetylchlorids mit dem Acrypnitril in Gegenwart eines organischen Lösungsmittels vornimmt.

3. Ein Verfahren nach Anspruch 1 oder 2, dadurch gekennzeichnet, dass man als Katalysator Eisen(II)chlorid, Eisen(III)chlorid, Eisenpulver, Ruthenium(III)chlorid, Ruthenium(II)dichloro-tris-triphenylphosphin, Kupferpulver, Kupferbronze, Kupfer(I)chlorid, Kupfer(II)chlorid, Kupfer(I)bromid, Kupfer(II)bromid, Kupfer(I)jodid, Kupfer(II)acetat, Kupfer(II)acetylacetonat, Kupfer(II)benzoylacetonat, Kupfer(II)sulfat, Kupfer(II)nitrat oder Kupfer(I)cyanid verwendet.

4. Ein Verfahren nach Anspruch 3, dadurch gekennzeichnet, dass man als Katalysator Kupferpulver, Kupferbronze, Kupfer(I)chlorid, Kupfer(II)chlorid, Kupfer(I)bromid, Kupfer(II)bromid, Kupfer(I)jodid oder Gemische davon verwendet.

5. Ein Verfahren nach einem der Ansprüche 1—4, dadurch gekennzeichnet, dass man die Umsetzung bei einer Temperatur zwischen etwa 70 und 220°C vornimmt.

6. Ein Verfahren nach einem der Ansprüche 1—5, dadurch gekennzeichnet, dass man die Umsetzung in geschlossenem System bei einem der jeweiligen Reaktionstemperatur entsprechenden Druck vornimmt.

7. Ein Verfahren nach einem der Ansprüche 1—6, dadurch gekennzeichnet, dass man als organische Lösungsmittel Alkylnitrile mit 2—5 C-Atomen oder 3-Alkoxypropionitrile mit 1 oder 2 C—Atomen im Alkoxyteil verwendet.

8. Ein Verfahren nach Anspruch 7, dadurch gekennzeichnet, dass man als organisches Lösungsmittel Acetonitril, Butyronitril, 3-Methoxypropionitril, und Acrylnitril verwendet.

9. Ein Verfahren nach Anspruch 1, dadurch gekennzeichnet, dass man das Reaktionsgemisch einer Nachbehandlung mit HCl oder einem HCl-abspaltenden Mittel unterwirft.

10. Ein Verfahren nach Anspruch 1, dadurch gekennzeichnet, dass man das Reaktionsgemisch einer Nachbehandlung mit einem Hydroxylionen liefernden Mittel unterwirft.

## Claims

1. A process for the preparation of 2,3,5,6-tetrachloropyridine and/or 3,5,6-trichloropyridin-2-ol, which process comprises reacting trichloroacetyl chloride with acrylonitrile in the presence of a metal of the main group VIII or of the auxiliary groups VIa, VIIa, Ib and IIb of the Periodic Table, or a compound of such a metal, as catalyst.

2. A process according to claim 1, wherein the reaction of trichloroacetyl chloride with acrylonitrile is performed in the presence of an organic solvent.

3. A process according to either claim 1 or claim 2, wherein the catalyst used is selected from the group consisting of: iron(II) chloride, iron(III) chloride, iron powder, ruthenium(III) chloride, ruthenium(II) dichloro-tris-triphenylphosphine, copper powder, copper bronze, copper(I) chloride, copper(II) chloride, copper(I) bromide, copper(II) bromide, copper(I) iodide, copper(II) acetate, copper(II)acetylacetonate, copper(II) benzoylacetonate, copper(II) sulfate, copper(II) nitrate or copper(I) cyanide.

4. A process according to claim 3, wherein the catalyst used is selected from the group consisting of: copper powder, copper bronze, copper(I) chloride, copper(II) chloride, copper(I) bromide, copper(II) bromide or copper(I) iodide, or a mixture thereof.

5. A process according to any one of claims 1—4, wherein the reaction is performed at a temperature of between about 70 and 220°C.

6. A process according to any one of claims 1—5, wherein the reaction is performed in a closed system at a pressure corresponding to the respective reaction temperature.

7. A process according to any one of claims 1—6, wherein the organic solvent used is an alkyl nitrile having 2—5 carbon atoms or a 3-alkoxypropionitrile having 1 or 2 carbon atoms in the alkoxy moiety.

8. A process according to claim 7, wherein the organic solvent used is selected from the group consisting of: acetonitrile, butyronitrile, 3-methoxypropionitrile and acrylonitrile.

9. A process according to claim 1, wherein the reaction mixture is subjected to an aftertreatment with HCl or with a dehydrochlorinating agent.

10. A process according to claim 1, wherein the reaction mixture is subjected to an aftertreatment with a hydroxyl ion donor.

## Revendications

1. Procédé de préparation de 2,3,5,6-tétrachloropyridine et/ou de 3,5,6-trichloropyridin-2-ol, caractérisé en ce qu'on fait réagir du chlorure de trichloracétyle en présence d'un métal du groupe principal VIII ou des sous-groupes VIa, VIIa, Ib et IIb de la classification périodique des éléments ou d'un composé d'un tel métal comme catalyseur avec de l'acrylonitrile.

2. Procédé selon la revendication 1, caractérisé en ce qu'on conduit la réaction du chlorure de trichloracétyle avec l'acrylonitrile en présence d'un solvant organique.

3. Procédé selon les revendications 1 et 2, caractérisé en ce qu'on utilise comme catalyseur le chlorure de fer(II), le chlorure de fer(III), la poudre de fer, le chlorure de ruthénium(III), la dichloro-tris-triphénylphosphine de ruthénium(II), la poudre de cuivre, les alliages de cuivre et de bronze, le chlorure de cuivre(I), le chlorure de cuivre(II), le bromure de cuivre(I), le bromure de cuivre(II), l'iodure de cuivre(I), l'acétate de cuivre(II), l'acétylacétonate de cuivre(II), le benzoylacétonate de cuivre(II), le sulfate de cuivre(II), le nitrate de cuivre(II) ou le cyanure de cuivre(I).

4. Procédé selon la revendication 3, caractérisé en ce qu'on utilise comme catalyseur la poudre de cuivre, les alliages de cuivre et de bronze, le chlorure de cuivre(I), le chlorure de cuivre(II), le bromure de cuivre(I), le bromure de cuivre(II), l'iodure de cuivre(I) ou leurs mélanges.

5. Procédé selon l'une des revendications 1—4, caractérisé en ce qu'on conduit la réaction à une température comprise entre environ 70 et 220°C.

6. Procédé selon l'une des revendications 1—5, caractérisé en ce qu'on conduit la réaction en système fermé à une pression correspondant à la température réactionnelle considérée.

7. Procédé selon l'une des revendications 1—6, caractérisé en ce qu'on utilise comme solvants organiques des alcoylnitriles en $C_2$ à $C_5$ ou des 3-alcoxypropionitriles en $C_1$ ou $C_2$ dans la partie alcoxy.

8. Procédé selon la revendication 7, caractérisé en ce qu'on utilise comme solvant organique l'acétonitrile, le butyronitrile, le 3-méthoxypropionitrile et l'acrylonitrile.

9. Procédé selon la revendication 1, caractérisé en ce qu'on soumet le mélange réactionnel à un traitement ultérieur avec HCl ou un agent séparant HCl.

10. Procédé selon la revendication 1, caractérisé en ce qu'on soumet le mélange réactionnel à un traitement ultérieur avec un agent donnant des ions hydroxyle.